# EUROPEAN PATENT APPLICATION

(11) **EP 4 063 856 A1**
(43) Date of publication of application: **28.09.2022**
(21) Application number: 21164819.1
(22) Date of filing: 25.03.2021
(51) Int. Cl.: G01N 33/50, G01N 33/52, C12Q 1/37

(54) **METHODS AND COMPOSITIONS FOR IN VITRO SCREENING OF PROTEASE INHIBITORS**

(71) Applicant: Universiteit Antwerpen, 2000 Antwerpen (BE)
(72) Inventor: De Meester, ingrid, 2610 Antwerp (BE)
(74) Representative: De Clercq & Partners

(57) **Abstract**

The application discloses *in vitro* screening methods for evaluating the effect of a compound on a protease, in particular for identifying a compound as a protease inhibitor. In particular, the methods of the invention comprise providing a composition comprising extracellular vesicle material, contacting said composition with the compound in the presence of a substrate of the protease, and determining the effect of the compound on the protease by assessing the enzymatic activity of the composition on the substrate. Further, the use of extracellular vesicles for the identification of a compounds as protease inhibitors is provided, as well as an *in vitro* screening kit for identifying a compound as a protease inhibitor.

## Description

### FIELD OF THE INVENTION

The invention is broadly in the medical field, and provides a novel *in vitro* screening method based on extracellular vesicles for evaluating and identifying compounds as enzyme inhibitors, such as transmembrane protease S1 member 2 (TMPRSS2) inhibitors. Also provided are the use of extracellular vesicles and an *in vitro* screening kit for the evaluation and identification of compounds as enzyme inhibitors.

### BACKGROUND OF THE INVENTION

Proteases in general have an important role in many signalling pathways and therefore represent interesting drug targets for a wide range of diseases. Although inhibitors of well-established protease targets have shown substantial therapeutic success, developing drugs for new protease targets has proved challenging in recent years. In addition, *in vitro* evaluation of potential protease targeting compounds might be hampered by the fact that some proteases to be used in such *in vitro* inhibitor screening assays are difficult to produce through recombinant methods. Although recombinant proteins can be produced at large scale using different types of expression systems such as *E. coli,* yeast cells and fungi, or mammalian cells, a major problem in the production of some recombinant proteins or enzymes is the lack of or difference in posttranslational processing, such as glycosylation, thereby potentially leading to issues with protein folding and/or subsequent activation. In turn, this results in less active or even inactive proteins and enzymes, thereby making them useless in activity-based applications, such as *in vitro* screening assays to identify protein inhibitors. An example of a protein that is hallmarked by a loss of activity when produced recombinantly is the human cell surface enzyme transmembrane protease serine S1 member 2 (TMPRSS2). Although recombinant TMPRSS2 is commercially available, the recombinant form only shows a very low specific activity or even no activity (Shrimp et al. 2020).

The human TMPRSS2 plays a prominent role in the infectivity of human corona- (CoV) and influenzaviruses (InV) (Shulla at al. J Virol 2011; Limburg et al, J Virol 2019), and hence forms one of the interesting therapeutic targets. During human InV and CoV infections, TMPRSS2 mediates the proteolytic processing of viral proteins required for host cell attachment. Furthermore, TMPRSS2 priming and processing has also been described to be involved in the tropism and pathogenesis of other human respiratory viruses. TMPRSS2 is thus an important player at the interface between humans and their viruses, and as such rightfully deserves a significant amount of attention as a promising therapeutic target to tackle viral infections.

### SUMMARY

The present inventors have addressed the challenges for the use of fully active proteins and enzymes in different applications such as in *in vitro* screening assays to evaluate potential protein and enzyme inhibitors. Although recombinant proteins can be produced at large scale using different types of expression systems, common problems are still encountered when recombinant proteins and enzymes are applied in *in vitro* screening assays. The lack of or differences in posttranslational processing of said proteins and enzymes results in less active or even inactive proteins and enzymes, such that their activity is limited, making them less suitable for certain applications, such as in *in vitro* screening assays. Present inventors discovered that enzymes, such as proteases, present as constituting components of intact extracellular vesicles retain their full activity and are concentrated therein such that these extracellular vesicles can be applied in different applications such as *in vitro* screening and identification of novel candidate compounds as inhibitors against said enzymes. More specifically, the inventors have established that intact extracellular vesicles or lysates thereof can be used in *in vitro* screening assays to evaluate and search compounds directed to transmembrane protease S1 member 2 (TMPRSS2), such as TMPRSS2 inhibitors.

Accordingly, a first aspect of the invention provides the use of extracellular vesicles in *in vitro* screening methods for compounds which affect enzyme activity, particularly enzyme inhibitors, in particular protease inhibitors, even more in particular TMPRSS2 inhibitors. In particular embodiments, the invention provides an *in vitro* screening method for evaluation of the effect of a compound on the activity of an enzyme on its substrate, in particular a protease, the method comprising providing a composition comprising extracellular vesicle material from a biological sample, contacting said composition with the compound in the presence of a substrate of said enzyme, in particular said protease, and determining the effect of said compound on the activity of said enzyme, in particular the protease. In further embodiments, the *in vitro* method is a method of identifying a compound as an enzyme inhibitor, in particular as a protease inhibitor, wherein the effect of the compound on the enzyme, in particular on the protease, is evaluated by assessing the enzymatic activity of said composition on the substrate, wherein inhibition of said enzymatic activity on the substrate identifies the compound as an enzyme inhibitor, in particular as a protease inhibitor.

In particular embodiments, the invention provides an *in vitro* method for evaluation of the effect of a compound against TMPRSS2, the method comprising providing a composition comprising extracellular vesicle material from a biological sample of one or more subjects, contacting said composition with the compound against TMPRSS2 in the presence of a substrate of TMPRSS2, and determining the effect of said compound on TMPRSS2. In further embodiments, the *in vitro* method is a method of identifying a compound as a TMPRSS2 inhibitor wherein the effect of the compound on TMPRSS2 is evaluated by assessing the enzymatic activity of said composition on the substrate, wherein inhibition of said enzymatic activity on the substrate identifies the compound as a TMPRSS2 inhibitor.

In particular embodiments, the method comprises providing a composition comprising extracellular vesicle material and contacting said composition with the compound against a protease, such as TMPRSS2. In particular embodiments, the extracellular vesicle material comprises intact extracellular vesicle material. In further embodiments, the extracellular vesicle material comprises lysed extracellular vesicle material. In particular embodiments, the methods encompass providing intact extracellular vesicles from a biological sample of one or more subjects, and optionally preparing a lysate from the extracellular vesicles. In particular embodiments, the methods comprise, contacting the composition comprising extracellular vesicle material with the compound in the presence of a substrate of the protease, in particular TMPRSS2, and evaluating the effect of the compound on the protease by assessing the enzymatic activity of said composition comprising the extracellular vesicle material on the substrate in the presence of the compound, wherein inhibition of said enzymatic activity on the substrate identifies the compound as a protease inhibitor, in particular as a TMPRSS2 inhibitor.

In particular embodiments, the biological sample is a bodily fluid sample; preferably the biological sample is a seminal fluid sample.

In particular embodiments, the substrate is conjugated to a detectable label; preferably to a detectable label that becomes detectable when the protease, in particular TMPRSS2, activates or cleaves the substrate.

In particular embodiments, the *in vitro* screening method of the present invention is a high throughput screening method.

A further aspect provides an *in vitro* screening kit for evaluation of the effect of a compound against a protease, in particular against TMPRSS2. In certain embodiments, said *in vitro* screening kit comprises a composition comprising extracellular vesicle material from a biological sample of one or more subjects. The *in vitro* screening kit further comprises a substrate of the protease, in particular a substrate of TMPRSS2. The *in vitro* screening kit may further comprise a control compound with known effect on the protease, in particular against TMPRSS2. The *in vitro* screening kit may further comprise means, such as buffers, for evaluating the activity of the protease, in particular TMPRSS2, on the substrate. In a further embodiment, the invention provides an *in vitro* screening kit for identifying a compound as TMPRSS2 inhibitor, said *in vitro* screening kit comprising: a composition comprising extracellular vesicle material that comprises TMPRSS2, and optionally a substrate of TMPRSS2, a control compound with known potency against TMPRSS2, and/or means, such as buffers, for evaluating the activity of TMPRSS2 on the substrate.

### BRIEF DESCRIPTION OF DRAWINGS

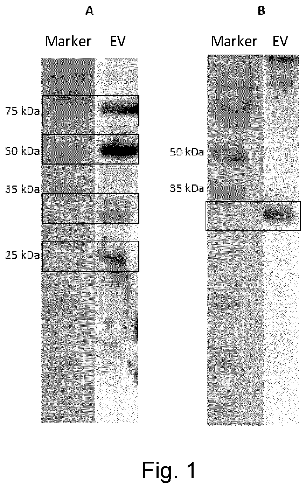

**Fig. 1** **Confirmation of the presence of TMPRSS2 in extracellular vesicles from seminal fluid.** A) Western blot of extracellular vesicles (EV) obtained from a seminal fluid sample with anti-TMPRSS2 ab: The bands at 75 and 50 kDa represent the full-length TMPRSS2 with and without glycosylation. The bands at lower molecular weight are the serine protease domain of TMPRSS2 with and without glycosylation. B) Electrophoresis and blot on a 12% gel of EVs after incubation with FP-biotin to visualize serine proteases in the EV suspensions.

### DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Recombinant proteins are nowadays frequently used in cellular and molecular biology applications. Nevertheless, recombinant protein expression and purification for *in vitro* studies can be a real challenge and still requires huge investments in both time and costs in order to obtain high yields of pure and active proteins. Although recombinant proteins can be produced at large scale using different types of expression systems such as *E. coli,* yeast cells and fungi, or animal cells, common problems are still encountered. A major problem in the production of some recombinant proteins or enzymes is the lack of proper protein folding and/or posttranslational processing, leading to less active or even inactive proteins and enzymes. After all, protein folding is a complex process that contributes to the full activity of the recombinant protein. Absent or inadequate protein folding results in misfolded recombinant proteins often with an impaired or even absent activity, thereby making them useless in activity-based applications, such as *in vitro* screening assays to identify protein inhibitors. An example of such a protein that is less active when produced recombinantly is the human cell surface enzyme TMPRSS2. Although recombinant TMPRSS2 is commercially available, it only shows a very low specific activity. More specifically, commercially available recombinant TMPRSS2 proteins expressed in HEK293, *E. coli,* and *in vitro* wheat germ constructs have not shown activity, and the yeast-expressed recombinant TMPRSS2 at a concentration of 1 µM only showed a maximum of 27% substrate conversion after 60 minutes, indicating its impaired activity (Shrimp et al. 2020). On the other hand, the demand for active proteases such as the TMPRSS2 protein in biotechnological applications is high since proteases, and hence also TMPRSS2, form an important therapeutic target against which novel candidate compounds with potential protease inhibiting activity have to be tested *in vitro* before they can proceed to further *in vivo* testing.

With the present invention, and as further shown in the experimental section, the inventors discovered that extracellular vesicles form an important source of proteases, such as TMPRSS2, that have retained their activity. More specifically, they found that extracellular vesicles obtained from a seminal fluid sample, also referred herein as prostasomes, contain high levels of active proteases such as TMPRSS2 and can be used in an *in vitro* screening method for evaluating and/or identifying compounds for their effect on proteases, such as TMPRSS2.

The present invention thus provides an *in vitro* method for evaluating and/or identifying the effect of a compound on an enzyme, more specifically against a protease. Said method comprises providing a composition comprising extracellular vesicle material from a biological sample of one or more subjects, contacting said composition with the compound in the presence of a substrate of the enzyme, in particular a substrate of the protease, and determining the effect of said compound on the enzyme, in particular on the protease. In preferred embodiments, the protease is TMPRSS2 and the *in vitro* method is for identifying and/or evaluating the effect of the compound on TMPRSS2. In certain embodiments, the effect of the compound on the protease, in particular TMPRSS2, is evaluated by assessing the enzymatic activity of the composition comprising extracellular vesicle material on the substrate, wherein inhibition of said enzymatic activity on the substrate identifies the compound as a protease inhibitor, in particular as a TMPRSS2 inhibitor.

As used herein, the term "extracellular vesicles" includes, but is not limited, to all vesicles released from the cells by any mechanism, therefore including secreted and exocytosed extracellular vesicles, thereby encompassing exosomes, but also including vesicles released by exocytosis, reverse budding, fission of membrane(s), and release of apoptotic bodies and hybrid vesicles containing acrosomal and sperm plasma membrane components. As used herein, the term "extracellular vesicle material" comprises any material derived from extracellular vesicles. Extracellular vesicle material can thus comprise intact extracellular vesicles, a lysate of extracellular vesicles, or a mixture thereof. It can also include any other material that is derived from extracellular vesicles.

In the context of this invention, the extracellular vesicles are isolated from a biological sample of one or more subjects. In some embodiments, the biological sample is obtained from one subject. In some other embodiments, the biological sample is obtained from several subjects, such that the biological sample is a pool or mixture of multiple biological samples obtained from multiple subjects. The biological sample obtained from the one or more subjects can be any bodily fluid. For example, the biological sample can be peripheral blood, sera, plasma, ascites, urine, cerebrospinal fluid, sputum, saliva, bone marrow, synovial fluid, amniotic fluid, cerumen, breast milk, bronchoalveolar lavage fluid, seminal fluid (including prostate fluid), Cowper's fluid or pre-ejaculatory fluid, female ejaculate, sweat, faeces, tears, cyst fluid, pleural or peritoneal fluid, pericardial fluid, lymph, chyme, chyle, bile, interstitial fluid, menses, pus, sebum, vomit, vaginal secretions, mucosal secretions, stool water, pancreatic juice, lavage fluid from sinus cavities, bronchopulmonary aspirates or other lavage fluids. In further embodiments, the biological sample is selected from seminal fluid, Cowper's fluid or pre-ejaculatory fluid or bronchoalveolar lavage fluid. Even more preferably, the biological sample is seminal fluid. In some embodiments, the biological sample is thus a combination or a mixture of seminal fluid samples obtained from multiple subjects.

The biological sample can be collected according to any method known in the art. In certain embodiments, the methods comprise the step of, for example, providing and/or collecting the biological sample, in particular the ejaculate or seminal fluid from the subject using a collection device pre-equilibrated to a temperature at or below the body temperature of the subject.

Seminal fluid forms an important source of extracellular vesicles derived from the prostate gland. Extracellular vesicles present in seminal fluid are also referred to as prostasomes, aposomes or seminosomes. The extracellular vesicles found in seminal fluid are membrane-bound storage vesicles originating from prostate epithelial cells. The extracellular vesicles in seminal fluid are membrane-bound vesicles with a diameter of 40-500 nm. They are secreted from the prostate gland either by exocytosis in which the extracellular vesicles bind to and fuse with the plasma membrane thereby releasing into the acinar lumen, or by translocation of the vesicles from the interior of the cell to the acinar lumen. Proteomic analysis of extracellular vesicles present in seminal fluid previously showed the presence of several different types of proteins, such as enzymes, transport or structural proteins, GTP proteins, chaperone proteins, signal transduction proteins, and unannotated proteins (Utleg et al., The prostate, 2003).

The methods of the present invention comprise providing a composition comprising extracellular vesicle material. In particular embodiments the composition comprising extracellular vesicle material comprises intact extracellular vesicles. In particular embodiments the composition comprising extracellular vesicle material comprises a lysate of extracellular vesicles. In particular embodiments, the method thus encompass providing intact extracellular vesicles from a biological sample of one or more subjects, and optionally preparing a lysate from the extracellular vesicles. Thus, in some embodiments the composition comprising extracellular vesicle material comprises intact extracellular vesicles from the biological sample. In some other embodiments, the composition comprising extracellular vesicle material comprises a lysate from extracellular vesicles. In still some other embodiments, the composition comprising extracellular vesicle material comprises both intact extracellular vesicles and a lysate of extracellular vesicles.

In certain embodiments, the composition comprising the extracellular vesicle material is prepared from the biological sample. In some embodiments, preparation of the composition comprising the extracellular vesicles from the biological sample includes a step of incubating the biological sample, such as for example wherein the incubating step includes controlling the temperature of the sample at a temperature in the range of about 4°C to the body temperature of the subject, preferably at a temperature in the range of about 4°C to about 18°C. In certain additional embodiments, the biological sample can be stored at a temperature of about -80°C to about 4°C before isolation of the extracellular vesicles takes place. In certain embodiments, and when the biological sample has been stored at a temperature below 0°C, the biological sample be thawed at about 4°C at the moment the extracellular vesicles are isolated from the biological sample.

The extracellular vesicles can be isolated or purified from the biological sample by methods known in the art, such as for example centrifugation methods in combination with size-exclusion chromatography and filtration methods. In certain embodiments, extracellular vesicles are isolated using ultracentrifugation in combination with size-exclusion chromatography. In certain embodiments, and when the biological sample is a seminal fluid sample, the seminal fluid can be centrifuged to pellet and remove the spermatozoa, followed by collection and standard centrifugation of the supernatant to remove dead cells and cellular debris. Extracellular vesicles can be further isolated using ultracentrifugation at a g force range in between about 80,000 x g and about 120,000 x g, followed by size-exclusion chromatography and an additional step of ultracentrifugation.

As shown in the examples, the inventors were able to isolate and purify intact extracellular vesicles from the biological sample, such as a seminal fluid sample, and were able to observe significant enzymatic activity therein. More specifically, the extracellular vesicles isolated from the seminal fluid were found to have significant trypsin-like activity. In certain embodiments, the *in vitro* methods therefore comprise providing a composition comprising intact extracellular vesicles wherein the extracellular vesicles are isolated from a biological sample of one or more subjects. In particular embodiments, the composition comprising extracellular vesicles comprises at least 80%, preferably at least 90%, more particularly at least 95% intact extracellular vesicles.

In certain embodiments, the isolated extracellular vesicles can be stored at a temperature range of about -80°C to about 4°C. In some preferred embodiments, the extracellular vesicles can be stored at a temperature of about -80°C for long-term storage. In some other embodiments, the extracellular vesicles can be stored at a temperature of about 4°C for a period up to 3 weeks. Accordingly, in particular embodiments, the methods provided herein comprise a step of storing the composition comprising extracellular vesicle material, in particular the composition comprising the intact extracellular vesicles at a temperature range of about -80°C to about 4°C.

In some embodiments, the composition comprising extracellular material comprises a lysate from the extracellular vesicles or a mixture of intact extracellular vesicles and a lysate of extracellular vesicles. The lysate can be prepared after isolation of the extracellular vesicles from the biological sample. In other embodiments, the lysate can be prepared simultaneously with the isolation of the extracellular vesicles from the biological material. The lysate can be prepared by any known method in the art using a standard lysis protocol to obtain a protein solution. For example, the lysate can be prepared using a known lysis buffer comprising Tris-SDS, Tris-Tween, Tris-octylgycoside, Tris-Triton, Guanidinium Chloride, or urea-thiourea.

In the methods of the present invention, the composition comprising extracellular vesicle material is contacted with a compound in the presence of a substrate of an enzyme or protease present in the composition, thereby identifying whether the compound exerts any effect on the enzyme or protease. In certain embodiments, the compound is identified as an inhibitor of an enzyme such as a protease that is present in the composition comprising extracellular vesicle material. In other embodiments, the compound is identified as an enhancer of an enzyme such as a protease present in the composition comprising the extracellular vesicle material. In other embodiments, the compound is identified as a compound that does not specifically inhibits or enhances the activity of the enzyme such as a protease. As used herein, proteases, also referred to as proteolytic enzymes or proteinases, are enzymes that catalyse or increase the rate of proteolysis or the breakdown of proteins into smaller polypeptides or single amino acids. Enzymes which have been identified in prostasomes include serine proteases, aspartic proteases, metalloproteinases, or aminopeptidases. In particular embodiments, the serine proteases are selected from type II transmembrane serine proteases (TMPRSSs) such as TMPRSS2, or kallikreins such as kallikrein 3. In particular embodiments, the enzyme is a metalloproteinases including disintegrin and metalloproteinases, such as ADAMTS and ADAMS or neprilysin; or matrixmetalloproteinases (MMPs). Aspartic proteases can include gastricsin. Carboxypeptidases can include glutamate carboxypeptidase. Aminopeptidases can be selected from dipeptidyl peptidase IV (DPP IV), aminopeptidase N, X-prolyl aminopeptidase.

In the methods of the invention, the composition comprising the extracellular vesicle material is contacted with a compound with potential effect on the activity of an enzyme or protease to be evaluated in the presence of a substrate for said enzyme or protease, whereafter the effect or potency of the compound against said enzyme or protease is evaluated by assessing the enzymatic activity of said composition on the substrate. When referring to "effect", "potency" or "activity" of a compound "on/against an enzyme", herein it is intended to refer to the ability of the compound to inhibit or enhance the activity of said enzyme on a substrate. In particular embodiments the enzyme is a protease and the enzymatic activity of the enzyme is protease or proteolytic activity. Inhibition by the compound of said enzymatic activity on the substrate will then identify the compound as an inhibitor against the enzyme, whereas enhancement of the enzymatic activity on the substrate will identify the compound as an enhancer of the enzyme. For example, in an *in vitro* method for identifying a compound as a TMPRSS2 inhibitor, the composition comprising extracellular vesicle material will be contacted with said compound in the presence of a substrate of TMPRSS2, whereafter the effect of the compound on TMPRSS2 activity will be evaluated by assessing the enzymatic activity of said composition on the TMPRSS2 substrate. Observation of inhibition of said enzymatic activity on the substrate will then identify the candidate compound as a TMPRSS2 inhibitor.

The selection of the substrate in the methods of the invention is determined by the enzyme of interest as well as the method of detection envisaged as will be detailed herein below. Within the context of the methods of the present invention, the substrate is preferably contacted with the vesicles in a solution. Where the enzyme is a protease, the solution may comprise a purified or substantially purified recombinant substrate, such as a polypeptide, in a suitable buffer. The solution may also comprise a synthetic substrate polypeptide or a peptide derivative in a suitable buffer. The substrate can also be provided immobilized to a platform or a surface. The substrate may be immobilized such that cleavage products derived from it are retained or released from the surface. If the contacting between the composition comprising the extracellular vesicle material and the compound in the presence of the substrate takes place in solution or on a surface or platform, the method is carried out under conditions that allow the enzyme to function. Suitable conditions include, but are not limited to room temperature (such as 25°C) to 37°C, a buffer comprising Tris-HCI, Hepes, or phosphate at a pH in the range of pH 7-9 and containing no to moderate amounts of CaCl₂.

The present invention provides *in vitro* methods for evaluation of the effect of a compound on an enzyme, in particular a protease. In particular embodiments, the methods of the invention encompass determining the inhibitory or enhancing effect of a compound on a protease, such as TMPRSS2, DPP IV or aminopeptidase N. In a particular embodiment, the compound is evaluated for its TMPRSS2-inhibiting activity and said compound is contacted with a composition comprising extracellular vesicle material in the presence of a substrate of TMPRSS2. In another particular embodiment, the compound is evaluated for its DPP IV inhibiting activity and said compound is contacted with a composition comprising extracellular vesicle material in the presence of a substrate of DPP IV. In still another embodiment, the compound is evaluated for its aminopeptidase N inhibiting activity and said compound is contacted with a composition comprising extracellular vesicle material in the presence of a substrate of aminopeptidase N. In some embodiments, the compound is a compound with an unknown activity against any of the enzymes, in particular proteases, in the composition comprising extracellular vesicle material, and the enzyme inhibiting or enhancing effect or potency is evaluated in the *in vitro* screening methods according to the invention by contacting the compound with the vesicle material and one or more substrates.

In the methods of the present invention, the composition comprising the extracellular vesicle material, the compound and the substrate can be contacted in any order. The composition comprising the extracellular vesicle material may be contacted first with the compound and then with the substrate. This type of pre-incubation may be necessary to allow sufficient time for a compound to have an effect on the enzymatic activity of the extracellular vesicle material. The composition with the extracellular vesicle material may be contacted first with the substrate and then with the compound. This order is useful for determining how quickly the compound can exert its effect on composition comprising the extracellular vesicle material. The composition with the extracellular vesicle material may be contacted with the substrate and the compound at the same time. Contacting is carried out for a sufficient period to allow the enzymatic activity on the substrate to be measured by the methods described below. The composition comprising the extracellular vesicle material is preferably contacted with the substrate in the presence of the compound.

The composition with the extracellular vesicle material and the substrate are contacted in a manner that allows a physical interaction between the composition comprising the extracellular vesicle material and the substrate. This is necessary for the enzyme to act on the substrate, e.g. for the protease to cleave the polypeptide substrate. However, it should be understood that the compound may interfere with the physical interaction of the enzyme with the substrate, e.g. interfere with the cleavage of the substrate by irreversibly binding to the active site of the protease or by effects on protease stability. Under such circumstances, there may be no interaction between the enzyme, particularly the protease and the substrate.

The composition comprising extracellular vesicle material and the candidate compound are contacted in any manner that allows the candidate compound to have an effect on the enzymatic activity. Preferably, the candidate compound is contacted with the extracellular vesicle material and the candidate compound in solution.

The *in vitro* screening methods or kits disclosed herein are of particular interest for evaluating the effect of a compound against an enzyme, such as a protease or for identifying a compound as an enzyme inhibitor, such as a protease inhibitor, in particular as a TMPRSS2 inhibitor. Any compound can be used as a compound in the different aspects of the invention. The compound(s) are preferably compounds that are suspected of affecting, and preferably inhibiting, enzymatic activity, more particularly protease activity, such as TMPRSS2 protease activity. The compound(s) can however also be compounds that are suspected of enhancing protease activity, such as TMPRSS2 activity. The compounds can be provided in any suitable form. They are typically in solution. The solution typically comprises a suitable buffer or solvent composition. The compounds may be any chemical or biological compounds used in drug screening programmes. They may be natural or synthetic.

In particular embodiments of the methods of the present invention, in order to allow for evaluating the effect of a compound on an enzyme, more particularly on a protease such as TMPRSS2, or for identifying a compound as an enzyme modulator, such as an enzyme inhibitor, more particularly a protease inhibitor, such as a TMPRSS2 inhibitor, a comparison can be made with a control, such as with a control value for the enzymatic activity of said composition comprising extracellular vesicle material. The enzyme activity, such as protease activity, observed following the contacting of the composition comprising extracellular vesicle material with the compound and the substrate is typically expressed as a value and can be compared with the control value. The control value is the enzymatic activity, more particularly the protease activity observed under conditions where the composition comprising extracellular vesicle material has been contacted with the substrate, but has not been contacted with the compound. Preferably, the conditions are otherwise identical to those used to determine the enzymatic activity value, more particularly the protease activity value, upon contacting the composition comprising extracellular vesicles with the compound. Following the comparison with the control, preferably with a control value, the effect of the compound may be identified in terms of a decrease or increase in enzymatic activity, more particularly the protease activity, with respect to the control value. In some embodiments, the effect of the compound may be identified in terms of an increase in enzymatic activity, particularly protease activity with respect to the control value. An increase in protease activity is indicative of an enhancer of the protease activity. A decrease in enzymatic activity, particularly protease activity is indicative of an inhibitor of the protease activity.

Preferably, the control value is obtained while carrying out the method of the invention in the absence of a compound having any effect on the enzyme or the composition comprising extracellular vesicle material, which is considered as a control reaction. For example, a control reaction is performed at the same time as reaction(s) where the composition comprising extracellular vesicle material is contacted with the substrate and the compound. This ensures that the control value is obtained under the same conditions as the protease activity measured following contacting of composition comprising extracellular material with the substrate and the compound. The control value can also be obtained separately from the detection of activity of the compound. For instance, the control value may be obtained beforehand and recorded, for instance on a computer. The control value may be used for multiple repetitions of the method when testing different compounds. The control value can be derived from more than one control reaction. For instance, the control value may be the arithmetic mean of the measurements obtained from several, such as 2, 5, 10, 15, or more, control reactions. In order to allow for an effective comparison, the control value has the same units as the measurement in the test sample with which it is being compared. A person skilled in the art is capable of obtaining such a value.

The type of control value referred to above is commonly known in the art as a "negative control". The method of the invention can also be carried out in conjunction with one or more positive controls for the protease activity of the composition comprising extracellular vesicle material. This involves carrying out reactions using one or more compounds which are known enhancers or inhibitors of the enzymatic activity under consideration. A positive control allows for validation or measurement of the enzymatic activity, particularly the protease activity, that is used in the method of the invention. For instance, this may be useful to allow comparison of results that have been obtained using different sources of extracellular vesicles comprising the enzyme, in particular the protease. A positive control also allows the extent to which the candidate compound enhances or inhibits the enzymatic activity, particularly the protease activity, to be determined. Known TMRPSS2 inhibitors that can function as positive control compounds can include, but are not limited to, nafamostat or camostat, or any derivatives thereof. Known DPP IV inhibitors that can function as positive control compounds can include, but are not limited to, sitagliptin, vildagliptin, or linaliptin, or any derivatives thereof. Known aminopeptidase N inhibitors that can function as positive control compounds can include, but are not limited to, bestatin, or any derivatives thereof.

The incubation period of the reaction constituents in the methods of the present invention prior to measurement of enzymatic activity, particularly protease activity, will be selected on the basis of the time required to generate a signal of appropriate strength. Measurement of enzymatic activity, particularly protease activity, can be performed at one or more timepoints following contacting the composition with extracellular vesicle material with the compound. This may allow for a determination of the duration and stability of the effect of the compound. Similarly, enzymatic activity, particularly protease activity, can be measured at one or more timepoints subsequent to the addition of the substrate to allow for determination of the effects of the compound on the kinetics of the enzymatic activity, particularly the protease activity. As discussed above, the substrate can be contacted with the composition comprising the extracellular vesicle material prior to contacting with the compound. This may allow for a determination of how quickly the compound exerts its effect on pre-existing enzymatic activity, particularly protease activity, of the extracellular vesicle material.

Techniques for measuring enzymatic activity, particularly protease activity, are well known in the art. Any technique may be used. The method preferably involves detecting one or more specific cleavage products derived from the substrate. In some embodiments, the substrate is conjugated to a detectable label. Preferred methods of measuring enzymatic activity, particularly protease activity, involve fluorescence, an immunoassay or mass spectrometry. In a most preferred embodiment, enzymatic activity, particularly protease activity, is measured by fluorescence, and the substrate is conjugated to a fluorescent label that becomes detectable when the enzyme, in particular the protease cleaves the substrate. Measuring substrate cleavage using fluorescence is well known in the art. For example, a substrate may be labelled with a fluorogenic moiety and cleavage can be monitored by a change in the fluorescence spectrum or a decay in the fluorescent signal. Examples of fluorogenic moieties that can act as detectable labels include, but are not limited to, coumarins, fluoresceins, rhodamins, resorufins, and demethylacridinones, 4-methoxy-2 naphthylamines. In a preferred embodiment, the fluorogenic moiety is a coumarin. In a particularly preferred embodiment, the coumarin is 7-amino-4-methylcoumarin (AMC).

A preferred fluorescence-based method that may be used to measure enzymatic activity, particularly protease activity, is using kinetic or endpoint fluorescence measurement, wherein the fluorescent signal of the cleaved substrate is kinetically measured over time or at the endpoint of the reaction. Also fluorescence resonance energy transfer (FRET) can be used to measure the enzymatic activity, in particular the protease activity. FRET uses two fluorophores, a donor and an acceptor. Excitation of the donor by an energy source (e.g. flash lamp or fluorometer laser) triggers an energy transfer to the acceptor if they are within a given proximity of each other. The acceptor in turn emits light at its given wavelength. The use of long-lived fluorophores combined with time-resolved detection (a delay between excitation and emission detection) is preferred to minimize interfered.

Another preferred method is based on the use of chromogenic substrates using kinetic or endpoint methods.

Measuring substrate cleavage using an immunoassay is also well-known in the art. The immunoassay can involve specific detection of one or more cleavage products derived from the substrate. Conversely, an immunoassay can be used to detect clearance or degradation of the substrate by measuring the amount of uncleaved substrate remaining after the action of the enzyme, in particular the protease on the substrate. Any suitable immunoassay which allows for detection of cleavage products or uncleaved substrate by an antibody may be used. Any suitable commercially available antibody for a given target may be used. A preferred immunoassay is enzyme-linked immunosorbent assay (ELISA).

Measuring substrate cleavage using mass spectrometry is also well known in the art. Cleavage products derived by the action of the enzyme, in particular the protease on the substrate can be separated on the basis of their mass and charge to allow for a determination of the relative proportions of each specific cleavage product in the reaction mixture. In such embodiments, the reaction mixture may be concentration prior to analysis by use of a suitable antibody which precipitates all cleavage products.

In certain embodiments, the substrate is conjugated to a detectable label. The label can by any moiety known in the art, including chromogenic enzyme systems, such as horseradish peroxidase, alkaline phosphatase, β-galactosidase, glucose 6-phosphate dehydrogenase. These enzyme labels are reacted with a chromogenic substrate that can be detected by conventional techniques. In an embodiment, the label is horseradish peroxidase, and the chromogenic substrate for detection is tetramethylbenzidine. The label can also be a fluorogenic moiety. Examples of fluorogenic moieties that can act as detectable labels include, but are not limited to, coumarins, fluoresceins, rhodamins, resorufins, demethylacridinones and 4-methoxy-2 naphthylamines In a preferred embodiment, the fluorogenic moiety is a coumarin. In a particularly preferred embodiment, the coumarin is 7-amino-4-methylcoumarin (AMC).

In a preferred embodiment of the invention, an *in vitro* screening method is provided to identify a compound as a TMPRSS2 inhibitor. TMPRSS2 is a serine protease with trypsin-like activity and able to cleave its substrate after Arginine or Lysine. Substrates for TMPRSS2 can therefore be any peptide substrates comprising Arginine or Lysine. In certain embodiments, substrates for TMPRSS2 are peptides selected from Boc-Leu-Gly-Arg , Boc-Gln-Ala-Arg, Ac-Val-Arg-Pro-Arg (SEQ ID No: 1), Cbz-Gly-Gly-Arg, Cbz-_{D}Arg-Pro-Arg or Cbz-_{D}Arg-Gly-Arg wherein Boc is a ter-butyloxycarbonyl group, Ac is an acetylated group and Cbz is a benzyloxycarbonyl group that form protecting groups during synthesis of the substrates (Shrimp et al. 2020). In some further embodiments, said substrates are conjugated to a detectable label or detectable moiety; preferably a fluorogenic moiety such as AMC. Thus in some specific embodiments, the substrate is selected from Boc-Leu-Gly-Arg-AMC, Boc-Gln-Ala-Arg-AMC, Ac-Val-Arg-Pro-Arg-AMC, Cbz-Gly-Gly-Arg-AMC, Cbz-_{D}Arg-Pro-Arg-AMC or Cbz-_{D}Arg-Gly-Arg-AMC. In some further preferred embodiment, the TMPRSS2 substrate is Boc-Gln-Ala-Arg-AMC, also referred to as Boc-QAR-AMC. Typically for all these substrates is the release of the detectable moiety or label, such as AMC, after contact with active TMPRSS2, which enables detection and quantification of the TMPRSS2 protease activity on the substrate.

In certain embodiments, the *in vitro* screening methods as disclosed herein are high throughput screening methods. In some embodiments the high throughput *in vitro* screening methods are performed on a large scale with high throughput by incorporating, e.g. an array-based assay system and at least one automated or semi-automated step. For example, the methods can be set up using multi-well plates in which the composition comprising the extracellular vesicle material, the candidate compound(s) and the substrate are dispensed and/or added in individual wells in a systematic manner using a multiwell delivery device suited to the geometry of the multiwell plate. Manual and robotic multiwell devices suitable for use in a high throughput screening assay are well known by those skilled in the art. Each well or array can be mapped in a one-to-one manner to a particular test condition, such as the candidate compound. Readouts can also be performed in this multiwell array; preferably using a multiwell plate reader device or the like. Examples of such devices are well known in the at and are available through commercial sources. Sample and reagent handling can be automated to further enhance the throughput capacity of the *in vitro* screening method, such that dozens, hundreds, thousands, or even millions of parallel assays can be performed in a day or in a week. Fully robotic systems are known in the art.

The present invention further provides the use of extracellular vesicles for evaluating the effect of a compound on an enzyme, in particular on a protease such as TMPRSS2, or for the identification of compounds as enzyme inhibitors, such as protease inhibitors. In a preferred embodiment, the use of extracellular vesicles is provided for the identification of compounds as TMPRSS2 inhibitors. In said use, the extracellular vesicles are provided in a composition with extracellular vesicle material as disclosed herein. The extracellular vesicle material may comprise intact extracellular vesicles, a lysate of extracellular vesicles or a combination thereof. In some further embodiments, the identification of candidate compounds as protease inhibitors is performed by any of the *in vitro* screening methods as disclosed herein.

In another aspect, the invention provides an *in vitro* screening kit for evaluating the effect of a compound on an enzyme, in particular on a protease, such as TMPRSS2, or for identifying a compound as a protease inhibitor such as a TMPRSS2 inhibitor. Said *in vitro* screening kit comprises a composition comprising extracellular vesicle material from a biological sample of one or more subjects. In a further embodiment, the extracellular vesicle material comprises intact extracellular vesicles. In another further embodiment the extracellular vesicle material comprises lysed extracellular vesicles. The composition comprising the extracellular vesicle material can thus comprise intact extracellular vesicles, a lysate of extracellular vesicles or a mixture thereof. In a preferred embodiment, the composition comprising the extracellular vesicle material comprises intact extracellular vesicles. The *in vitro* screening kit optionally also comprises a substrate of an enzyme, in particular a protease; preferably a substrate of TMPRSS2. In another embodiment, the *in vitro* screening kit further comprises a control compound with known effect or potency on a protease, such as TMPRSS2. In still some other embodiments, the *in vitro* screening kit comprises means for evaluating the activity of the enzyme, in particular the protease, even more in particular TMPRSS2, on the substrate, such as buffers, or any other compounds for evaluating the cleavage of the substrate by the enzyme, in particular by the protease.

In another aspect, the *in vitro* screening kit comprises a control compound with a known effect or potency on the enzyme, in particular the protease, a substrate for the enzyme, in particular the protease and means such as buffers for evaluating the enzymatic activity, in particular the protease activity on the substrate in the presence of the control compound and one or more compounds. Said compound(s) can be optionally provided in the *in vitro* screening kit. The compound(s) can also be provided by the user of the *in vitro* screening kit. Optionally, the *in vitro* screening kit comprises a composition comprising extracellular vesicle material that comprises an enzyme, in particular a protease, wherein said extracellular vesicle material is isolated from a biological sample of one or more subjects.

The methods of the present invention involve the use of extracellular vesicle material obtained from biological samples collected from one or more subjects. Typically, the one or more subject in any of the uses, methods or kits of the present invention is human. More specifically, the subject is a male human. However, the subject may also be non-human. For instance, the subject can be a commercially farmed animal, such as a horse, cow, sheep or pig, or may be a pet such as a cat or dog. Preferred non-human animals include, but are not limited to, primates, such as a monkey. In particular embodiments, the subject is a mammal. In some embodiments, the biological sample is obtained from one subject. In some other embodiments, the biological sample is obtained from several subjects, such that the biological sample is a pool or mixture of multiple biological samples obtained from multiple subjects. In some preferred embodiments, the biological sample is a mixture of multiple biological samples from multiple human subjects. Though, in some other preferred embodiments, the biological sample can also be obtained from a single human subject.

It is apparent that there have been provided in accordance with the invention products, methods, and uses, that provide for substantial advantages as set forth above. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The above aspects and embodiments are further supported by the following non-limiting examples.

### EXAMPLES

### Materials and methods

### Prostosome isolation

Prostasomes or extracellular vesicles were isolated from semen samples using ultracentrifugation and size-exclusion chromatography. Samples were thawed at 4°C, pooled, and centrifuged for 10 minutes at 900 x g to pellet spermatozoa. The supernatant was collected and centrifuged for 10 minutes at 2000 x g. Afterwards, the collected supernatant was subjected to centrifugation for 30 minutes at 10 000 x g to remove dead cells and cellular debris. Subsequently, the resulting supernatant was ultracentrifuged at 100 500 x g for 120 minutes (Optima XPN-100 Ultracentrifuge from Beckman Coulter) to pellet the prostasomes. Finally, prostasomes were washed with 20 mM Tris-HCI pH 7.4 and resuspended in the same buffer. This protocol was adapted from Carlsson et al. and De Meester et al.

The obtained suspension was purified on a Superdex 200 column equilibrated with 20 mM Tris-HCI pH 7.4. Fractions with high DPPIV were pooled and centrifuged for 145 minutes at 200 000 x g (Optima Max-XP from Beckman Coulter) to obtain pure prostasome pellets. The resuspended pellets were stored at -80°C. DPPIV activity was measured with 1 mM Gly-Pro-pNA (Bachem) in 50 mM Tris-HCI pH 8.3 at 405 nm and 37°C on a Tecan M200 multi-plate reader.

### Prostasome characterization

The sizes of the extracellular vesicles present in the prostasome suspensions were determined with Zetaview Twin (Particle Metrix). Zetaview Twin combined with CellMask Green labeling was also used to determine the concentration of the extracellular vesicles. (These experiments were executed at VITO, Vlaamse Instelling voor Technologisch Onderzoek). The protein content was estimated using a Bradford assay.

Trypsin-like activity in the intact prostasomes was determined using 100 µM Boc-QAR-AMC (Bachem) in 50 mM Tris 120 mM NaCl pH 8,0. Fluorescence was measured kinetically at 37°C for 20 minutes on a Tecan F200 multi-plate reader at an excitation and emission wavelength of 380 nm and 465 nm, respectively. The stability of the trypsin-like activity at 4°C was assessed by measuring the activity in three different prostasome preparations 1, 3, 8, 21 and 30 days after thawing.

The presence of TMPRSS2 in the prostasome suspension was confirmed using a western blot with an anti-TMPRSS2 antibody sc-101847 (Santa Cruz Biotechnology) detection. To observe the presence of other serine proteases in the suspension, the prostasomes were incubated with FP-biotin (Santa Cruz Biotechnology) for 30 minutes at 37°C. Afterwards, the samples were boiled and loaded on a 12% bisacrylamide gel for electrophoresis. FP-biotin labels active serine proteases so they can be visualised using streptavidin-HRP (R&D Systems) and SuperSignal^{™} West Femto Maximum Sensitivity Substrate (ThermoFisher Scientific).

### Recombinant TMPRSS2

The expression vector, encoding the serine protease domain of TMPRSS2, a His-tag and a secretion signal, was transformed into TOP10F' *E. coli* cells. The cells were incubated in LB-medium containing 100 µg/mL ampicillin overnight at 37°C and plasmids were prepared using a Plasmid kit from Qiagen. HEK293T cells were seeded in a T75 flask at a density of 8x10⁶ cells in DMEM GlutaMAX^{™} (Gibco^{™}) containing 10% fetal bovine serum and 1% penicillin and streptomycin. The cells were incubated overnight at 37°C. The next day, when the cells were 80-90% confluent, the medium was removed and replaced by Opti- MEM^{™} (Gibco^{™}). DNA was mixed with polyethyleneimine in a 1/1.5 ratio in Opti-MEM^{™} (Gibco^{™}) and added to the flasks. Cells were again incubated at 37°C for 3 days. Cell medium containing the TMPRSS2 serine protease domain was collected 4 days post-transfection and trypsin-like activity was determined as described above. Besides the activity measurement, a western blot with an anti-Histidine Tag antibody (Bio-Rad) was performed to confirm the success of the expression.

### The use of prostasomes in an assay for inhibitor screening

Two known TMPRSS2 inhibitors, camostat mesylate and nafamostat mesylate (Sigma-Aldrich), were screened for their inhibitory potency in two assays. On the one hand, intact prostasomes and on the other hand, medium containing recombinant TMPRSS2 (rTMPRSS2) were used. The prostasome suspension and rTMPRSS2 were diluted so that less than 10% substrate conversion was observed after 30 minutes of measurement and trypsin-like activities of both preparations were similar (approximately 0.025 U/L). The inhibitors were added to the samples in concentrations of 10 µM, 1 µM and 100 nM, followed by 15 minutes of incubation at 37°C. The substrate, Boc-QAR-AMC (Bachem), was added in a final concentration of 30 µM. This concentration was determined to be the Michaelis-Menten constant for Boc-QAR-AMC and recombinant TMPRSS2. Fluorescence was measured kinetically at 37°C for 20 minutes on a Tecan F200 multi-plate reader at an excitation and emission wavelength of 380 nm and 465 nm, respectively. The inhibitory effect was calculated by dividing the trypsin-like activity without inhibitor by the activity with inhibitor and was expressed as percentage inhibition. We are currently optimising the assay for high throughput screening and IC50 determinations of nafamostate and camostat are also ongoing.

### Results

### Prostosome characterization

Zetasview Twin experiments showed a heterogenous group of vesicles between 95 and 283 nm and a mean vesicle diameter of 179 nm. These results corroborate the literature and could also be confirmed with electron microscopy images. The vesicle concentration varied between 2,1x10¹³ and 5.7x10¹³ vesicles per mL and the protein content ranged between 27 and 35 g/L.

### TMPRSS2 in prostasomes

Unexpectedly, a high and very stable trypsin-like activity was measured in the suspension of intact prostasomes. An activity of 100-300 U/L was measured in prostasomes compared to an activity of 0.04 U/L in the medium containing recombinant TMPRSS2. Furthermore, the activity in the prostasome suspensions remains stable for several months at -80°C and 1 month at 4°C.

Figure 1 (A) shows a western blot with an anti-TMPRSS2 antibody to confirm the described presence of TMPRSS2 in the prostasome preparation. The bands at 75 kDa and 54 kDa represent the full-length TMPRSS2 with and without glycosylation, respectively. The bands at approximately 32 kDa and 25 kDa derive from the TMPRSS2 serine protease domain with and without glycosylation. The western blot after incubation with FP-biotin is shown in Figure 1 (B). It shows that FP-biotin labels the accessible active site of TMPRSS2 and this is the most abundant serine protease in the prostasome suspensions.

### Prostasomes in an assay for inhibitor screening

The prostasome suspension was compared with the medium containing recombinant TMPRSS2 in an assay for the inhibitor screening of camostat and nafamostat. Less than 4x10⁹ vesicles were needed to obtain a signal where S/B was above 25 and this vesicle concentration will be further optimised. The compounds had a similar inhibitory effect on both samples, but inhibition of rTMPRSS2 was slightly less efficient. For example, 100 nM camostat inhibited 99% of the activity in prostasomes but only 73% of recombinant TMPRSS2. All inhibitory effects are shown in Table 1, where they are expressed as the percentage inhibition of trypsin-like activity.

The findings mentioned here indicate that efficient inhibitors will not be missed when prostasomes are used as a screening tool for TMPRSS2-inhibitors. Identified 'hits' should be confirmed in an activity measurement with recombinant TMPRSS2.

**Table 1. The inhibitory effect of nafamostat and camostat (10 µM, 1 µM and 100 nM) on the trypsin-like activity in prostasomes and medium with recombinant TMPRSS2 was determined. The inhibitory effect at each compound concentration is shown as the percentage of inhibited trypsin-like activity.**

| **Inhibitory effect (%):** | **Nafamostat** | | **Camostat** | |
|---|---|---|---|---|
| | Prostasomes | rTMPRSS2 | Prostasomes | rTMPRSS2 |
| **10 µM** | 100 | 99 | 100 | 100 |
| **1 µM** | 100 | 99 | 100 | 88 |
| **100 nM** | 100 | 89 | 99 | 73 |

### References

Carlsson L, Nilsson O, Larsson A, Stridsberg M, Sahlén G, Ronquist G. Characteristics of human prostasomes isolated from three different sources. Prostate. 2003 Mar 1;54(4):322-30.
De Meester I, Vanhoof G, Lambeir AM, Scharpé S. Use of immobilized adenosine deaminase (EC 3.5.4.4) for the rapid purification of native human CD26/dipeptidyl peptidase IV (EC 3.4.14.5). J Immunol Methods. 1996 Jan 16;189(1):99-105.
Limburg H, Harbig A, Bestle D, Stein DA, Moulton HM, Jaeger J, Janga H, Hardes K, Koepke J, Schulte L, Koczulla AR, Schmeck B, Klenk HD, Böttcher-Friebertshäuser E. TMPRSS2 is the major activating protease of Influenza A virus in primary human airway cells and Influenza B virus in human Type II pneumocytes. J Virol 2019 Oct 15;93(21):e00649-19.
Shrimp JH, Kales SC, Sanderson PE, Simeonov A, Shen M, Hall MD. An Enzymatic TMPRSS2 Assay for Assessment of Clinical Candidates and Discovery of Inhibitors as Potential Treatment of COVID-19. ACS Pharmacol Transl Sci. 2020 Oct 9;3(5):997-1007.
Shulla A, Heald-Sargent T, Subramaya G, Zhao J, Perlman S, Gallagher T. A transmembrane serine protease is linked to the severe acute respiratory syndrome coronavirus receptor and activates virus entry. J Virol 2011 Jan;85(2):873-82.
Utleg AG, Yi EC, Xie T, Shannon P, White JT, Goodlett DR, Hood L and Lin B. Proteomic analysis of human prostasomes. The Prostate 2003; 56:150-161.

## Claims

1. An *in vitro* method for evaluation of the effect of a compound on TMPRSS2 activity, the method comprising:
- providing a composition comprising extracellular vesicle material from a biological sample of one or more subjects;
- contacting said composition with the compound in the presence of a substrate of TMPRSS2; and
- determining the effect of said compound on TMPRSS2.

2. The *in vitro* method of claim 1, which is a method of identifying a compound as a TMPRSS2 inhibitor wherein the effect of the compound on TMPRSS2 is evaluated by assessing the enzymatic activity of said composition on the substrate, wherein inhibition of said enzymatic activity on the substrate identifies the compound as a TMPRSS2 inhibitor.

3. The *in vitro* method of claim 1 or 2 wherein the extracellular vesicle material comprises intact extracellular vesicles.

4. The *in vitro* method of any of the preceding claims wherein the biological sample is a bodily fluid sample, such as a seminal fluid sample.

5. The *in vitro* method of any of the preceding claims wherein the one or more subjects is a mammal, preferably a human; even more preferably a male human.

6. The *in vitro* method of any of the preceding claims wherein the substrate is conjugated to a detectable moiety; preferably a fluorogenic moiety.

7. The *in vitro* method of claim 6 wherein the detectable moiety is a moiety that becomes detectable when TMPRSS2 cleaves the substrate.

8. The *in vitro* method of any of the preceding claims wherein the substrate is selected from Boc-Leu-Gly-Arg-AMC, Boc-Gln-Ala-Arg-AMC, Cbz-Gly-Gly-Arg-AMC, Ac-Val-Arg-Pro-Arg-AMC, Cbz-Gly-Gly-Arg-AMC Cbz-_{D}Arg-Pro-Arg-AMC or Cbz-_{D}Arg-Gly-Arg-AMC; preferably wherein the substrate is Boc-Gln-Ala-Arg-AMC.

9. The *in vitro* method according to any of the preceding claims wherein the *in vitro* screening method is a high throughput screening method.

10. Use of extracellular vesicles for the evaluation of the effect a compound on TMPRSS2 activity; in particular for the identification of a compound as a TMPRSS2 inhibitor.

11. An *in vitro* screening kit for evaluating the effect of a compound on TMPRSS2 activity or for identifying a compound as TMPRSS2 inhibitor, the *in vitro* screening kit comprising:
- a composition comprising extracellular vesicle material from a biological sample of one or more subjects;
- optionally, a substrate of TMPRS22;
- optionally, a control compound with known inhibitory effect on TMPRSS2;
- optionally, means (such as buffers) for evaluating the activity of TMRPSS2 on the substrate.

12. The *in vitro* screening kit of claim 11 wherein the extracellular vesicle material comprises intact extracellular vesicles.

13. The *in vitro* screening kit of claim 11 or 12 wherein the biological sample is a bodily fluid sample, such as a seminal fluid sample.

14. The *in vitro* screening kit of any one of claims 11 to 13, wherein the substrate is conjugated to a detectable moiety; preferably a fluorogenic moiety.

15. The *in vitro* screening kit of any one of claims 11 to 14, wherein the detectable moiety becomes detectable when the TMPRSS2 cleaves the substrate.


SEQUENCE LISTING

<110>  Universiteit Antwerpen

<120>  METHODS AND COMPOSITIONS FOR IN VITRO SCREENING OF PROTEASE
       INHIBITORS

<130>  UA-020-EP-PRIO1

<160>  1

<170>  PatentIn version 3.5

<210>  1
<211>  4
<212>  PRT
<213>  Artificial Sequence

<220>
<223>  Protease substrate



<220>
<221>  MOD_RES  for V (Val): acetylation
<222>  (1)..(1)

<400>  1

Val Arg Pro Arg
1